# EUROPÄISCHE PATENTANMELDUNG

(11) **EP 1 046 708 A1**
(43) Veröffentlichungstag der Anmeldung: **25.10.2000**
(21) Anmeldenummer: 00107916.9
(22) Anmeldetag: 13.04.2000
(51) Int. Cl.: C12N 15/31, C07K 14/32, G01N 33/68

(54) **Expression bakterieller Kaliumkanäle in eukaryontischen Wirtszellen und deren Verwendung zur Identifizierung von Antibiotika**

(30) Priorität: 23.04.1999 DE 19920044
(71) Anmelder: Forschungsgesellschaft Genion mbH, 20149 Hamburg (DE)
(72) Erfinder: Pongs, Olaf, Prof.Dr., 20249 Hamburg (DE)
(74) Vertreter: UEXKÜLL & STOLBERG

(57) **Zusammenfassung**

Gegenstand der Erfindung sind eukaryontische Expressionsvektoren, die die Expression bakterieller Kaliumkanäle der LCT B Familie ermöglichen. Ferner sind erfindungsgemäß diese Vektoren enthaltende Wirtszellen eingeschlossen, die die bakteriellen Kaliumkanäle exprimieren. Ferner wird erstmals ein Verfahren zum Identifizieren von Substanzen zur Verfügung gestellt, die bakterielle Kaliumkanäle öffnen, schließen, modulieren, aktivieren oder inaktivieren können und somit bakteriozide oder bakteriostatische Eigenschaften aufweisen. Gemäß einer besonderen Ausführungsform der Erfindung dient das Verfahren zum Identifizieren bzw. Auffinden von Antibiotika.

SEQUENZPROTOKOLL

<110> Forschungsgesellschaft GENION m.b.H.

<120> Expression bakterieller Kaliumkanaele in
      eukaryontischen Wirtszellen und deren Verwendung zur
      Identifizierung von Antibiotika

<130> P053366

<140>
<141>

<150> DE 199 20 044.0
<151> 1999-04-23

<160> 9

<170> PatentIn Ver. 2.1

<210> 1
<211> 405
<212> DNA
<213> Bacillus stearothermophilus

<220>
<221> CDS
<222> (1)..(402)

<400> 1
atg gat tac gca ttc ctc gga gtg gtg acc gcc gtg ttg ctc ggc agc     48
Met Asp Tyr Ala Phe Leu Gly Val Val Thr Ala Val Leu Leu Gly Ser
  1               5                  10                  15

atc act tcg ctt tgg aca gcg agc gta caa gca acg cac cgc ctc tcc     96
Ile Thr Ser Leu Trp Thr Ala Ser Val Gln Ala Thr His Arg Leu Ser
             20                  25                  30

ctt gac agt tta tgg gtg ctc gtt caa tgg tat ggg acg atg ctg cta    144
Leu Asp Ser Leu Trp Val Leu Val Gln Trp Tyr Gly Thr Met Leu Leu
         35                  40                  45

ggg ttt gcg atg att tat atg att ttg caa gcc aac ggc cat cat gta    192
Gly Phe Ala Met Ile Tyr Met Ile Leu Gln Ala Asn Gly His His Val
     50                  55                  60

ttt act ccg tca cca agc agt gca gca gga aac cgc ctg tct atg ctg    240
Phe Thr Pro Ser Pro Ser Ser Ala Ala Gly Asn Arg Leu Ser Met Leu
 65                  70                  75                  80

gaa gac agt cta tat tta agc ggc atg acc ctc ctg tcg gtc gga tac    288
Glu Asp Ser Leu Tyr Leu Ser Gly Met Thr Leu Leu Ser Val Gly Tyr
                 85                  90                  95

gga gac gtc acc ccg gtc ggc atc ggg cgg tgg atc gcc atc gcc gag    336
Gly Asp Val Thr Pro Val Gly Ile Gly Arg Trp Ile Ala Ile Ala Glu
            100                 105                 110

gcg ctg ctt ggc tac att atg cct gct gtt atc gtc acg cgc acg gta    384
Ala Leu Leu Gly Tyr Ile Met Pro Ala Val Ile Val Thr Arg Thr Val
        115                 120                 125

ttt gac tcg gat cgc cgt tga                                          405
Phe Asp Ser Asp Arg Arg
    130


<210> 2
<211> 134
<212> PRT
<213> Bacillus stearothermophilus

<400> 2
Met Asp Tyr Ala Phe Leu Gly Val Val Thr Ala Val Leu Leu Gly Ser
1               5                   10                  15

Ile Thr Ser Leu Trp Thr Ala Ser Val Gln Ala Thr His Arg Leu Ser
            20                  25                  30

Leu Asp Ser Leu Trp Val Leu Val Gln Trp Tyr Gly Thr Met Leu Leu
        35                  40                  45

Gly Phe Ala Met Ile Tyr Met Ile Leu Gln Ala Asn Gly His His Val
    50                  55                  60

Phe Thr Pro Ser Pro Ser Ser Ala Ala Gly Asn Arg Leu Ser Met Leu
65                  70                  75                  80

Glu Asp Ser Leu Tyr Leu Ser Gly Met Thr Leu Leu Ser Val Gly Tyr
                85                  90                  95

Gly Asp Val Thr Pro Val Gly Ile Gly Arg Trp Ile Ala Ile Ala Glu
            100                 105                 110

Ala Leu Leu Gly Tyr Ile Met Pro Ala Val Ile Val Thr Arg Thr Val
        115                 120                 125

Phe Asp Ser Asp Arg Arg
    130



<210> 3
<211> 709
<212> RNA
<213> Bacillus stearothermophilus

<220>
<221> misc_feature
<222> (94)..(99)
<223> BamHI site

<220>
<221> misc_feature
<222> (100)..(108)
<223> Kozak site

<220>
<221> CDS
<222> (109)..(510)

<220>
<221> misc_feature
<222> (514)..(519)
<223> XbaI site

<220>
<221> misc_feature
<222> (675)..(709)
<223> poly-A-tail

<400> 3
ggggcgaauu aauucgagcu cgguacccag cuugcuuguu cuuuuugcag aagcucagaa 60

uaaacgcuca acuuuggcag aucaauuccc cggggauccg ccgccacc aug gau uac 117
Met Asp Tyr
1

gca uuc cuc gga gug gug acc gcc gug uug cuc ggc agc auc acu ucg 165
Ala Phe Leu Gly Val Val Thr Ala Val Leu Leu Gly Ser Ile Thr Ser
    5                   10                  15

cuu ugg aca gcg agc gua caa gca acg cac cgc cuc ucc cuu gac agu 213
Leu Trp Thr Ala Ser Val Gln Ala Thr His Arg Leu Ser Leu Asp Ser
20                  25                  30                  35

uua ugg gug cuc guu caa ugg uau ggg acg aug cug cua ggg uuu gcg 261
Leu Trp Val Leu Val Gln Trp Tyr Gly Thr Met Leu Leu Gly Phe Ala
                40                  45                  50

aug auu uau aug auu uug caa gcc aac ggc cau cau gua uuu acu ccg 309
Met Ile Tyr Met Ile Leu Gln Ala Asn Gly His His Val Phe Thr Pro
            55                  60                  65

uca cca agc agu gca gca gga aac cgc cug ucu aug cug gaa gac agu 357
Ser Pro Ser Ser Ala Ala Gly Asn Arg Leu Ser Met Leu Glu Asp Ser
        70                  75                  80

cua uau uua agc ggc aug acc cuc cug ucg guc gga uac gga gac guc 405
Leu Tyr Leu Ser Gly Met Thr Leu Leu Ser Val Gly Tyr Gly Asp Val
    85                  90                  95

acc ccg guc ggc auc ggg cgg ugg auc gcc auc gcc gag gcg cug cuu 453
Thr Pro Val Gly Ile Gly Arg Trp Ile Ala Ile Ala Glu Ala Leu Leu
100                 105                 110                 115

ggc uac auu aug ccu gcu guu auc guc acg cgc acg gua uuu gac ucg 501
Gly Tyr Ile Met Pro Ala Val Ile Val Thr Arg Thr Val Phe Asp Ser
                120                 125                 130

gau cgc cgu ugaucuagag caagcuugau cugguuacca cuaaaccagc 550
Asp Arg Arg

cucaagaaca cccgaaugga gucucuaagc uacauaauac caacuuacac uuuacaaaau 610

guuguccccc aaaauguagc cauucguauc ugcuccuaau aaaaagaaag uuucuucaca 670

uucuaaaaaa aaaaaaaaaa aaaaaaaaaa aaaaaaaaa 709

<210> 4
<211> 134
<212> PRT
<213> Bacillus stearothermophilus

<400> 4
Met Asp Tyr Ala Phe Leu Gly Val Val Thr Ala Val Leu Leu Gly Ser
1               5                   10                  15

Ile Thr Ser Leu Trp Thr Ala Ser Val Gln Ala Thr His Arg Leu Ser
            20                  25                  30

Leu Asp Ser Leu Trp Val Leu Val Gln Trp Tyr Gly Thr Met Leu Leu
        35                  40                  45

Gly Phe Ala Met Ile Tyr Met Ile Leu Gln Ala Asn Gly His His Val
    50                  55                  60

Phe Thr Pro Ser Pro Ser Ser Ala Ala Gly Asn Arg Leu Ser Met Leu
65                  70                  75                  80

Glu Asp Ser Leu Tyr Leu Ser Gly Met Thr Leu Leu Ser Val Gly Tyr
                85                  90                  95

Gly Asp Val Thr Pro Val Gly Ile Gly Arg Trp Ile Ala Ile Ala Glu
            100                 105                 110

Ala Leu Leu Gly Tyr Ile Met Pro Ala Val Ile Val Thr Arg Thr Val
        115                 120                 125

Phe Asp Ser Asp Arg Arg
    130

<210> 5
<211> 195
<212> PRT
<213> Bacillus stearothermophilus

<400> 5
Met Gly His His His His His His His His His His Ser Ser Gly His
1               5                   10                  15

Ile Glu Gly Arg His Met Asp Tyr Ala Phe Leu Gly Val Val Thr Ala
            20                  25                  30

Val Leu Leu Gly Ser Ile Thr Ser Leu Trp Thr Ala Ser Val Gln Ala
        35                  40                  45

Thr His Arg Leu Ser Leu Asp Ser Leu Trp Val Leu Val Gln Trp Tyr
    50                  55                  60

Gly Thr Met Leu Leu Gly Phe Ala Met Ile Tyr Met Ile Leu Gln Ala
65                  70                  75                  80

Asn Gly His His Val Phe Thr Pro Ser Pro Ser Ser Ala Ala Gly Asn
                85                  90                  95

Arg Leu Ser Met Leu Glu Asp Ser Leu Tyr Leu Ser Gly Met Thr Leu
            100                 105                 110

Leu Ser Val Gly Tyr Gly Asp Val Thr Pro Val Gly Ile Gly Arg Trp
        115                 120                 125

Ile Ala Ile Ala Glu Ala Leu Leu Gly Tyr Ile Met Pro Ala Val Ile
    130                 135                 140

Val Thr Arg Thr Val Phe Asp Ser Asp Arg Arg Ser Asp Pro Asn Ser
145                 150                 155                 160

Ser Ser Val Asp Lys Leu Ala Ala Ala Leu Glu Ile Lys Arg Ala Ser
                165                 170                 175

Gln Pro Glu Leu Ala Pro Glu Asp Pro Glu Asp Val Glu His His His
            180                 185                 190

His His His
        195

<210> 6
<211> 32
<212> DNA
<213> Kuenstliche Sequenz

<220>
<223> Beschreibung der kuenstlichen Sequenz: PCR Primer
      LctB Nde 1625

<400> 6
cagaggtaca tatggattac gcattcctcg ga                                32

<210> 7
<211> 30
<212> DNA
<213> Kuenstliche Sequenz

<220>
<223> Beschreibung der kuenstlichen Sequenz: PCR Primer
      LctB Bam25

<400> 7
aaggatccga acggcgatcc gagtcaaata                                   30

<210> 8
<211> 36
<212> DNA
<213> Kuenstliche Sequenz

<220>
<223> Beschreibung der kuenstlichen Sequenz: PCR Primer
      sense

<400> 8
attggatccg ccgccaccat ggattacgca ttcctc                            36

<210> 9
<211> 25
<212> DNA
<213> Kuenstliche Sequenz

<220>
<223> Beschreibung der kuenstlichen Sequenz: PCR Primer
      antisense

<400> 9
ctctctagat caacggcgat ccgag                                        25

## Beschreibung

Gegenstand der Erfindung sind eukaryontische Expressionsvektoren sowie Ribonukleinsäuren (RNAs), welche die Expression bakterieller Kaliumkanäle der LCT B Familie ermöglichen. Ferner sind erfindungsgemäß eukaryontische Wirtszellen eingeschlossen, welche die bakteriellen Kaliumkanäle exprimieren. Ferner wird erstmals ein Verfahren zum Identifizieren von Substanzen zur Verfügung gestellt, die bakterielle Kaliumkanäle öffnen, schließen, modulieren, aktivieren oder inaktivieren können und somit bakteriozide oder bakteriostatische Eigenschaften aufweisen. Gemäß einer besonderen Ausführungsform der Erfindung dient das Verfahren zum Identifizieren bzw. Auffinden von Antibiotika.

Die Kontrolle der intrazellulären Kaliumkonzentration in Bakterien spielt eine wesentliche Rolle bei der Aufrechterhaltung des Turgors, der Regulation von Enzymaktivitäten, der Kontrolle des zytoplasmatischen pH-Werts und des Schwimmverhaltens von Bakterien (Bakker, E.P. (1993) Cell K⁺ and K⁺ transport systems in Prokaryotes. In Alkaki Cation Transport Systems in Prokaryotes. Bakker, E.P., ed., Boca Raton: CRC, 205-2252; Epstein, W. and Schultz, S.G. (1965) Cation transport in Escherichia coli V. Regulation of cation content. J. Gen. Physiol. 49, 221-234). Die Größe der intrazellulären Kaliumkonzentration ist in der Regel abhängig von der extrazellulären Kaliumkonzentration, vom osmotischen Druck des Wachstumsmediums bzw. des extrazellulären Raums und von der Aktivität von Kaliumtransportsystemen und -kanälen, die in die Bakterienmembran eingelagert sind (s.u.). Im allgemeinen ist die Kaliumkonzentrationen im extrazellulären Raum wesentlich niedriger als in der Zelle. Dies bedeutet, daß die Membranen einem Kaliumgradienten ausgesetzt sind. Kann dieser nicht aufrecht erhalten werden, sind Zellen nicht überlebensfähig und lysieren. Bakterielle Zellen besitzen deshalb ein umfangreiches Repertoire an Kaliumtransportsystemen und Kaliumaustauschsystemen. In vielen Bakterien sind Gene für Kaliumtransportsysteme identifiziert und charakterisiert. Bei Kaliumtransportsystemen wird Kalium unter Energieverbrauch durch die Bakterienmembran transportiert.

Über bakterielle Kaliumkanäle, die einen Transport von Kalium ohne Energieaufwand ermöglichen, ist hingegen nur sehr wenig bekannt. In eukaryontischen Zellen sind Kaliumkanäle aus α- und β-Untereinheiten aufgebaut (Pongs, O. (1992) Molecular biology of voltage-dependent potassium channels. Physiol. Rev. 72, S69-S88; Chandy, K.G. and Gutman G.A. (1995) Voltage-gated potassium channel genes. In Handbook of Receptors and Channels: Ligand and voltage-gated ion channels. North R.A., ed., Boca Raton: CRC 1, 1-71; Jan, L.Y. and Jan, Y.N. (1997) Cloned potassium channels from eukaryotes and prokaryotes. Annu. Rev. Neurosci. 20, 91-123). Sowohl die α-Untereinheiten als auch die β-Untereinheiten eukaryotischer Kaliumkanäle können strukturell verschieden sein, so daß eine große Vielzahl verschiedener Heteromultimere möglich ist. Die α-Untereinheiten sind membranintegrierte Proteine, während die β-Untereinheiten je nach Kaliumkanal entweder auch membranintegrierte oder aber zytoplasmatische Proteine sind (Jan, L.Y. and Jan, Y.N. (1997) Cloned potassium channeis from eukaryotes and prokaryotes. Annu. Rev. Neurosci. 20, 91-123). Die α-Untereinheiten enthalten in der Regel alle Strukturmerkmale, um in *in vitro* Expressionssystemen funktionelle Kaliumkanäle auszubilden. Die β-Untereinheiten sind Hilfsproteine. Alle bisher bekannten α-Untereinheiten haben ein gemeinsames Strukturmotiv, das deshalb auch Kaliumkanal-Signatursequenz genannt wird. Die Signatursequenz macht einen wesentlichen Teil der Pore (P) der Kaliumkanäle aus, die für den selektiven Ein/Ausstrom von Kaliumionen durch die geöffneten Kaliumkanäle verantwortlich ist. Im einfachsten Fall wird die P-Domäne mit ihrer Signatursequenz von zwei hydrophoben membrandurchspannenden Segmenten, M1 und M2, flankiert, wobei die P-Domäne von der extrazellulären Seite in die Membran eintaucht. Der Bereich zwischen den membrandurchspannenden Segmenten, der die P-Domäne umfaßt, wird als M1-M2-Linker-Region bezeichnet. Dieses einfache strukturelle Grundprinzip findet sich bei Kaliumkanälen, die einwärtsgleichrichtende Eigenschaften haben (Doupnik, C.A., Davidson, N. and Lester H.A. (1995) The inward rectifier potassium channel family. Curr. Opin. Neurobiol. 5, 268-277). Einwärtsgleichrichtend bedeutet, daß die Kanäle bei negativen Membranpotentialen Einwärtsströme wesentlich besser leiten als bei positiven Membranpotentialen Auswärtsströme (Hille, B. (1992) Ionic channels of excitable membranes. Sunderland, MA: Sinauer. 2^{nd}ed.).

Die meisten einwärtsgleichrichtenden Kaliumkanäle (K_{IR}) sind aus Untereinheiten aufgebaut, die das einfache M1-P-M2 Motiv enthalten. Das Schaltverhalten der K_{IR}-Kanäle wird häufig durch extrinsische Faktoren bestimmt. Dies können regulatorische Proteine oder auch Kationen wie z.B. Mg²⁺ und Polyamine sein (Doupnik, C.A., Davidson, N. and Lester H.A. (1995) The inward rectifier potassium channel family. Curr. Opin. Neurobiol. 5, 268-277; Nichols, C.G. and Lopatin, A.N. (1997) Inward rectifier potassium channels. Annu. Rev. Physiol. 59, 171-191).

Im Stand der Technik ist bislang nur ein bakterielles Gen bekannt, dessen Expressionsprodukt ein M1-P-M2 Motiv aufweist, das KcsA-Gen. Es wurde aus dem gram-positiven Bakterium Streptomyces lividans (S. lividans) isoliert (Doupnik, C.A., Davidson, N. and Lester H.A. (1995) The inward rectifier potassium channel family. Curr. Opin. Neurobiol. 5, 268-277; Schrempf, H., Schmidt, O., Kümmerlen, R., Hinnah, S., Müller, D., Betzler, M., Steinkamp, T., and Wagner, R. (1995) A prokaryotic potassium ion channel with two predicted transmembrane segments from Streptomyces lividans. EMBO J. 14, 5170-5178). Das KcsA-Protein wurde in *E.coli* artifiziell exprimiert. Mit dem gereinigten Protein wurden in Lipidbilayer-Experimenten funktionelle, pH-sensitive Kaliumkanäle erhalten (Cuello, L.G., Romero, J.G., Cortes, D.M., and Perozo, E. (1998) pH-Dependent gating in the Streptomyces lividans K⁺channel. Biochemistry 37, 3229-3236; Perozo, E., Cortes, D.M. and Cuello, L.G. (1998) Three-dimensional architecture and gating mechanism of a K⁺channel studied by EPR spectroscopy. Nature Struct. Biol. 5, 459-469). Allerdings ist es anderen Arbeitsgruppen, die ebenfalls das KcsA-Protein in *E.coli* exprimiert haben, nicht gelungen, das KcsA-Protein funktionell in eukaryontischen Zellen zu exprimieren (Cuello, L.G., Romero, J.G., Cortes, D.M., and Perozo, E. (1998) pH-Dependent gating in the Streptomyces lividans K⁺channel. Biochemistry 37, 3229-3236). Dies wurde damit begründet, daß eine funktionelle Expression eines bakteriellen Membranproteins in eukaryontischenen Zellen nicht zu erwarten sei, da das Protein wahrscheinlich von Ribosomen am endoplasmatischen Retikulum synthetisiert werden müßte und von dort über den veskulären Transport bis zur Plamamembran gelangen müßte. Bakterien besitzen diese Strukturen (endoplasmatisches Retikulum, Vesikeltransport) nicht. Es wurde deshalb damit gerechnet, daß bakteriellen Proteinen die passenden Sequenzsignale für den Transport in eukaryotischen Zellen fehlen würden.

Zur Charakterisierung und zum Verständnis der bakteriellen Kaliumkanäle ist es dennoch wünschenswert, diese in eukarytischen Zellen exprimieren und funktionell untersuchen zu können. Nur so lassen sich die physiologischen und biophysikalischen Eigenschaften dieser Kanäle untersuchen. Die Kenntnis der Eigenschaften wiederum ist Voraussetzung dafür, die Funktion der bakteriellen Kanäle zu modulieren und zu regulieren und damit ihre Rolle im bakteriellen Kaliumhaushalt entscheidend zu beeinflussen. Die Expression von bakteriellen Kaliumkanälen in eukaryontischen Zellen würde auch den Aufbau von Testsystemen ermöglichen, mit denen Stoffe entwickelt und gefunden werden können, die als spezifische Regulatoren (z.B. zum Öffnen oder Blockieren) bakterieller Kaliumkanäle fungieren können und somit antibiotisch wirksam sind. Diese Stoffe wären Mitglieder einer neuen Klasse von Antibiotika, da zur Zeit keine Stoffe bekannt sind, die spezifisch bakterielle Kaliumkanäle modulieren.

Aufgabe der vorliegenden Erfindung ist es daher, Mittel zur Verfügung zu stellen, mit deren Hilfe bakterielle Kaliumkanäle in eukaryontischen Zellen exprimiert werden können. Ferner sollen Verfahren zur Verfügung gestellt werden, mit denen bakterielle Kaliumkanäle in eukaryontischen Zellen exprimiert werden können. Ferner ist es Aufgabe der vorliegenden Erfindung, ein neues Testsystem zur Verfügung zu stellen, mit dessen Hilfe Stoffe auf ihre Eignung getestet werden können, bakterielle Kaliumkanäle zu öffnen, zu schließen, zu modulieren, zu aktivieren oder zu inaktivieren. Insbesondere soll das Testsystem dazu verwendet werden können, um auf einfache Weise gezielt Wirkstoffe daraufhin zu testen, ob sie durch die Veränderung der Aktivität der bakteriellen Kaliumkanäle antibiotische Wirkung haben.

Erfindungsgemäß wird die Aufgabe durch den Gegenstand der Ansprüche 1 bis 34 gelöst, insbesondere durch eukaryontische Wirtszellen, die einen einwärtsgleichrichtenden bakteriellen Kaliumlcanal der LCT B Familie exprimieren.

Im Rahmen der vorliegenden Erfindung hat es sich überraschenderweise gezeigt, daß bakterielle Kaliumkanäle aus der LCT B Familie in eukaryontischen Zellen funktionell exprimiert werden können.

Erfindungsgemäß wird daher ein eukaryontischer Expressionsvektor zur Verfügung gestellt, der mindestens eine Nukleotidsequenz, die für eine Kaliumkanaluntereinheit kodiert, in einer Anordnung enthält, die die funktionelle Expression in Eukaryonten ermöglicht, wobei die Nukleotidsequenz die in SEQ ID NO: 1 ("SEQ ID NO" entspricht der numerischen Kennzahl 〈400〉) dargestellte Sequenz umfaßt oder ein Homologes, eine Mutante, ein Derivat oder ein Fragment derselben ist. Die in SEQ ID NO: 1 dargestellte Nukleotidsequenz umfaßt das bakterielle *lct B*-Gen. Die erfindungsgemäß eingeschlossenen Homologe, Mutanten, Derivate oder Fragmente dieser Nukleotidsequenz sind dadurch gekennzeichnet, daß sie eine Nukleotidsequenz aufweisen, die einen Sequenzabschnitt umfaßt, der eine Homologie von mindestens 40 %, vorzugsweise von mindestens 60 % und besonders bevorzugt von mindestens 80 % zu dem von Nukleotid 172 bis Nukleotid 312 in SEQ ID NO: 1 dargestellten Sequenzabschnitt aufweist. Dieser Bereich entspricht der M1-M2-Linker-Region der LCT B Proteine. Die von der in SEQ ID NO: 1 dargestellten Sequenz abgeleiteten Nukleinsäuremoleküle kodieren für weitere Mitglieder der LCT B-Kaliumkanaluntereinheit-Familie.

Die vorliegende Erfindung betrifft sowohl die o.g. Nukleotidsequenzen als auch diese Nukleotidsequenzen enthaltende Expressionsvektoren.

Besonders bevorzugt ist die Nukleotidsequenz, die Bestandteil des erfindungsgemäßen eukaryontischen Expressionsvektors ist, bakteriellen Ursprungs, d.h. sie kodiert für eine bakterielle Kaliumkanaluntereinheit.

Der erfindungsgemäße eukaryontische Expressionsvektor ist erhältlich, indem die Nukleotidsequenz, die erfindungsgemäß für eine Kaliumkanaluntereinheit kodiert, in einen eukaryontischen Expressionsvektor kloniert wird. Die entsprechenden Methoden sind dem Fachmann geläufig (Sambrook, J., Fritsch, E.F. and Maniatis, T. (1989) Molecular Cloning: A Laboratory Manual, second edition; Cold Spring Harbor Laboratory Press. New York.). Der eukaryontische Expressionsvektor enthält erfindungsgemäß vorzugsweise weitere Nukleotidsequenzen, die die funktionelle Expression der für eine Kaliumkanaluntereinheit kodierenden Nukleotidsequenz in Eukaryonten ermöglichen bzw. beeinflussen. Dazu zählen unter anderem Promotor- und Enhancersequenzen, Spleißsignale, Polyadenylierungssignale und ribosomale Bindungsstellen.

Im Rahmen der vorliegenden Erfindung wurde überraschenderweise festgestellt, daß die durch das *lct B*-Gen kodierte bakterielle Kaliumkanaluntereinheit eine geringe Sequenzhomologie (kleiner 40%) zu anderen, bisher bekannten Kaliumkanaluntereinheiten aufweist. Im Vergleich zu den im Stand der Technik bekannten eukaryontischen Kaliumkanaluntereinheiten besitzt die erfindungsgemäße bakterielle Kaliumkanaluntereinheit unterschiedliche elektrophysiologische Eigenschaften. Somit ist die durch das *lct B*-Gen kodierte Kaliumkanaluntereinheit das erste bekannte Mitglied einer neuen Familie von Kaliumkanaluntereinheiten.

Die erfindungsgemäßen Nukleinsäuremoleküle sind erhältlich, indem aus der Nukleotidsequenz des *lct B*-Genes mittels dem Fachmann bekannter Methoden Sonden hergestellt werden, mit deren Hilfe Genbanken, die das Genom von Bakterienstämmen umfassen, durchsucht werden können (Sambrook, J., Fritsch, E.F. and Maniatis, T. (1989) Molecular Cloning: A Laboratory Manual, second edition; Cold Spring Harbor Laboratory Press. New York. Vorzugsweise kommen dabei Genbanken von Bakterienstämmen in Betracht, die für den Menschen pathogen sind. Dazu zählen unter anderem Chlamydomonas reinhardtii, Chlorella kessleri, Chlorella vulgaris, Acetobacter methanolicus, Bacillus megaterium, Chlostridium aceticum, Lactobacillus acidophilus, Staphylococcus simulans, Streptomyces diastaticus, Borrelia burgdorferi, Chlostridium botulinum, Haemophilus influenzae, Klebsiella pneumoniae, Mycobacterium avium subsp. paratuberculosis, Neisseria gonorrhoeae, Pantoea agglomerans, Staphylococcus aureus und Vibrio cholerae, sind aber nicht auf diese beschränkt. Eine weitere Methode zur Identifizierung von Homologen zum lct B-Gen beinhaltet, daß zunächst mittels dem Fachmann bekannte Methoden Antikörper gegen das LCT B-Protein hergestellt werden. Mit Hilfe dieser Antikörper können dann Expressionsbibliotheken durchsucht werden (Sambrook, J., Fritsch, E.F. and Maniatis, T. (1989) Molecular Cloning: A laboratory Manual, 2^{nd}ed; Cold Spring Harbor Laboratory Press. New York.; Harlow, E. and Lane, D. (1988) Antibodies - A Laboratoy Manual: Cold Spring Harbor Laboratory Press).

Die erfindungsgemäßen Homologe, Mutanten und Derivate sind außerdem erhältlich, indem die für das LCT B-Protein kodierende Nukleotidsequenz mittels dem Fachmann vertrauter molekularbiologischer Methoden abgewandelt wird. Zu diesen Methoden zählt unter anderem "site directed mutagenesis", die Erfindung ist aber nicht auf diese beschränkt. Ferner sind die erfindungsgemäßen Nukleinsäuren über eine *in vitro* Synthese erhältlich (Krieg, P.A. and Melton, D.A. (1984) Functional messenger RNAs are produced by SP6 in vitro transcription of cloned cDNAs. Nucl. Aci. Res. 12, 7057-70; Current Protocols in Molecular Biology, edited by Ausubel, F.M. et al., John Wiley and Sons, Inc., New York, USA (letzte aktualisierte Fassung Herbst 1998).

Gemäß einer bevorzugten Ausführungsform sind die erfindungsgemäßen Homologe, Mutanten und Derivate RNA-Moleküle.

Gegenstand der Erfindung ist ferner ein RNA-Molekül, das die Nukleotide 109 bis 510 aus SEQ ID NO: 3 oder die oben genannten RNA-Moleküle, welche erfindungsgemäßen Homologe, Mutanten und Derivate sind, zusammen mit für die Translation wichtigen Regulationssequenzen, vorzugsweise eine Kozak-Stelle, umfaßt.

Das erfindungsgemäße RNA-Molekül ist erhältlich, indem ein erfindungsgemäßer Expressionsvektor, der eine Bindungsstelle für eine RNA-Polymerase, vorzugsweise T7 oder SP6, umfaßt, mit der an diese Bindungstelle bindenden RNA-Polymerase unter Bedingungen in Kontakt gebracht wird, die das Ablesen der Sequenz durch die RNA-Polymerase ermöglichen, so daß das erfindungsgemäße RNA-Molekül hergestellt wird. Die dazugehörigen Techniken und Bedingungen sind dem Fachmann bekannt (Krieg, P.A. and Melton, D.A. (1984) Functional messenger RNAs are produced by SP6 in vitro transcription of cloned cDNAs. Nucl. Aci. Res. 12, 7057-70; Sambrook et al., a.a.O.).

Eine besonders bevorzugte Ausführungsform der Erfindung umfaßt ein RNA-Molekül, das die in SEQ ID NO:3 gezeigte Nukleotidsequenz umfaßt.

Erfindungsgemäß eingeschlossen ist ferner eine eukaryontische Wirtszelle, die mit dem erfindungsgemäßen Expressionsvektor oder mit der erfindungsgemäßen RNA transformiert ist. Die erfindungsgemäße eukaryontische Wirtszelle ist erhältlich, indem eine eukaryontische Zelle mittels dem Fachmann bekannter Methoden mit dem erfindungsgemäßen Expressionsvektor oder der erfindungsgemäßen RNA transformiert wird. Diese Methoden umfassen unter anderem Ca²⁺ abhängige Transformation, Elektroporation, Lipofektion, die Erfindung ist aber nicht auf diese beschränkt (Sambrook, J., Fritsch, E.F. and Maniatis, T. (1989) Molecular Cloning: A laboratory Manual, 2^{nd}ed; Cold Spring Harbor Laboratory Press. New York.; Current Protocolls in Molecular Biology, edited by Ausubel, F.M. et al., John Wiley and Sons, Inc., New York, USA (letzte aktualisierte Fassung Herbst 1998)).

Im Rahmen der vorliegenden Erfindung sind als Wirtszellen CHO-Zellen oder Xenopus-Oozyten bevorzugt. Weitere eukaryontische Zellen, die sich erfindungsgemäß dafür eignen, mit dem erfindungsgemäßen eukaryontischen Expressionsvektor transformiert zu werden, sind L1-Zellen, COS-Zellen und HEK 293-Zellen, die Erfindung ist aber nicht auf diese beschränkt.

Im Rahmen der vorliegenden Erfindung exprimiert die Wirtszelle vorzugsweise einen funktionellen bakteriellen Kaliumkanal, wobei dieser bakterielle Kaliumkanal gemäß einer besonderen Ausführungsform der Erfindung auf der Oberfläche der Wirtszelle exprimiert wird.

Besonders bevorzugt ist eine eukaryontische Wirtszelle, die den erfindungsgemäßen eukaryontischen Expressionsvektor enthält und die einen funktionellen bakteriellen Kaliumkanal exprimiert. Dabei ist gemäß einer besonders bevorzugten Ausführungsform der Erfindung die erfindungsgemäße eukaryontische Wirtszelle ein Xenopus-Oozyt, der mit einem erfindungsgemäßen RNA-Molekül oder mit einem erfindungsgemäßen eukaryontischen Expressionsvektor transfiziert ist, der die in SEQ ID NO: 1 dargestellte Sequenz enthält, und wobei die erfindungsgemäße eukaryontische Wirtszelle den von dieser Sequenz kodierten einwärtsgleichrichtenden Kaliumkanal auf ihrer Oberfläche exprimiert.

Erfindungsgemäß eingeschlossen ist ferner ein bakterieller Kaliumkanal, der erhältlich ist entweder durch Expression der erfindungsgemäßen RNA oder durch Expression eines Nukleinsäuremoleküls mit der in SEQ ID NO: 1 dargestellten Nukleotidsequenz oder durch Expression der erfindungsgemäßen Homologen, Mutanten, Derivate oder Fragmente mit der o.g. Homologie von mindestens 40%, vorzugsweise von mindestens 50% und besonders bevorzugt von mindestens 80% zu dem von Nukleotid 172 bis Nukleotid 312 in SEQ ID NO:1 dargestellten Sequenzabschnitt, der der M1-M2-Linker-Region der LCT B-Proteine entspricht. Die präparative Expression der bakteriellen LCT B Kanäle und die Herstellung in gereinigter solubilisierter Form macht es möglich, alternative Assay-Systeme, die auf spektroskopischen Methoden beruhen, für die Untersuchung der Bindung von Stoffen an diese Kanäle einzusetzen. Dabei kann die Bindung von radioaktiv markierten Stoffen direkt gemessen werden oder in kompetitiven Verdrängungsassays bestimmt werden. Ferner ist erfindungsgemäß eingeschlossen, das LctB-Protein so abzuwandeln, daß es mit einem Fluoreszenzfarbstoff markiert werden kann. Bindung von Substanzen an fluoreszenzmarkierte LctB-Kanäle führt zu einer Änderung der Fluoreszenzintensität, die in Fluoreszenzspektrometern gemessen werden kann.

Die Erfindung betrifft ferner ein Verfahren zur Expression eines bakteriellen Kaliumkanals, bei dem man die erfindungsgemäßen eukaryontische Wirtszellen, vorzugsweise Xenopus Oozyten, unter geeigneten Bedingungen, die die Expression des Kaliumkanals ermöglichen, kultiviert. Diese Bedingungen variieren je nach Art der eukaryontischen Wirtszelle, sie sind aber dem Fachmann bekannt (Current Protocolls in Molecular Biology, a.a.O.).

Gemäß einer bevorzugten Ausführungsform wird von den Wirtszellen ein einwärtsrektifizierender Kaliumkanal auf ihrer Oberfläche exprimiert werden.

Erfindungsgemäß eingeschlossen ist ferner die Verwendung der erfindungsgemäßen Wirtszellen zum Identifizieren und Testen von Substanzen, die geeignet sind, die von den erfindungsgemäßen Wirtszellen exprimierten Kaliumkanäle zu öffnen, zu schließen, zu aktivieren, zu inaktivieren und/oder in ihren biophysikalischen Eigenschaften zu verändern.

Erfindungsgemäß eingeschlossen ist ferner ein Verfahren zum Identifizieren und Testen von Substanzen, die geeignet sind, die von den erfindungsgemäßen Wirtszellen exprimierten Kaliumkanäle zu öffnen, zu schließen, zu aktivieren, zu inaktivieren und/oder in ihren biophysikalischen Eigenschaften zu verändern, bei dem man
a) an den erfindungsgemäßen Wirtszellen den Kaliumauswärtsstrom mißt,
b) die Wirtszellen mit einer zu untersuchenden Substanz in Kontakt bringt und
c) an den Wirtszellen erneut den Kaliumauswärtsstrom mißt,
wobei der Unterschied zwischen den Kaliumauswärtsströmen vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die hinzuzusetzende Konzentration der Substanz ist, um eine Änderung der Kaliumauswärtsströme zu erreichen.

Besonders bevorzugt exprimieren die verwendeten Wirtszellen den erfindungsgemäßen Kaliumkanal auf ihrer Oberfläche. Auf diese Weise können die Substanzen dadurch identifiziert bzw. getestet werden, daß man den Ausstrom von Ionen aus den Zellen durch den erfindungsgemäßen Kaliumkanal mißt. Das Ausströmen von Ionen wird bevorzugt mit der 'patch-clamp'-Methode (vgl. z.B. O.P. Hamill *et al., Pflügers Arch.* (1981) 85-100) durch Anlegen depolarisierender Testpotentiale bestimmt.

Im Rahmen der vorliegenden Erfindung ist ferner eingeschlossen, die erfindungsgemäßen Wirtszellen nach dem Fachmann gut bekannten Methoden mit ⁸⁶Rb-Ionen zu beladen, die durch Kaliumkanäle so gut wie Kaliumionen permeieren können. Die beladenen Zellen können in Gegenwart von zu testenden Substanzen kultiviert werden. Danach kann der Einfluß der Substanzen auf den ⁸⁶Rb-Auswärtsstrom der mit ⁸⁶Rb beladenen Zellen mit dem Fachmann bekannten Methoden gemessen werden (R.S. Rogowski *et al. Mol. Pharmacol.* **50** (1996) 1167-1177).

Erfindungsgemäß wird eine Substanz als eine öffnende Substanz bezeichnet, wenn nach Zugabe der Substanz bei einem Membranpotential, bei dem ohne Zugabe der Substanz keine Kaliumauswärtsströme fließen, Kaliumauswärtsstöme fließen.

Erfindungsgemäß wird eine Substanz als eine aktivierende Substanz bezeichnet, wenn nach Zugabe der Substanz ein bereits vorhandener Kaliumauswärtsstrom verstärkt wird.

Erfindungsgemäß wird eine Substanz als eine schließende Substanz bezeichnet, wenn nach Zugabe der Substanz bei einem Membranpotential, bei dem ohne Zugabe der Substanz Kaliumauswärtsströme fließen, keine Kaliumauswärtsströme fließen.

Erfindungsgemäß wird eine Substanz als eine inaktivierende Substanz bezeichnet, wenn nach Zugabe der Substanz ein bereits vorhandener Kaliumauswärtsstrom vermindert wird, ohne daß der Kaliumauswärtsstrom vollständig zum Erliegen kommt.

Erfindungsgemäß wird eine Substanz als eine verändernde Substanz bezeichnet, wenn durch Zugabe der Substanz biophysikalische Eigenschaften des Kaliumkanals wie Spannungsabhängigkeit, Leitfähigkeit, Aktivierungszeitkonstanten, Inaktivierungszeitkonstanten, Schaltverhalten, Offenzeiten oder Geschlossenzeiten verändert werden.

Änderungen in der Spannungsabhängigkeit der Aktivierung führen erwartungsgemäß bei Testpotentialen, die Kaliumauswärtsströme hervorrufen, zu einer Stromzunahme oder Stromabnahme. Leitfähigkeitsänderungen führen ebenfalls zu einer Zu- oder Abnahme der Kaliumauswärtsströme. Änderungen der Aktivierungszeitkonstanten führen zu einer Verlangsamung oder Beschleunigung der Aktivierung von Kaliumauswärtsströmen. Änderungen der Inaktivierungszeitkonstanten sowie des Schaltverhaltens können während eines Testpulses zu einer Zunahme oder Abnahme der Auswärtsströme führen. Das gleiche gilt, wenn die Offenzeiten oder Geschlossenzeiten der zu messenden Kaliumkanäle verändert werden (B. Hille, Ionic Channels of Excitable Membranes, 2. Ausgabe (1993), Sinauer Associates Inc., Sunderland, Massachusetts, USA).

Erfindungsgemäß wird eine Substanz auch dann als eine verändernde Substanz bezeichnet, wenn durch Zugabe der Substanz die Zelloberflächenexpression des Kaliumkanals verändert wird. Eine Veränderung der Zelloberflächenexpression führt zu einer Zunahme bzw. Abnahme der zu messenden Kaliumauswärtsströme.

Gegenstand der Erfindung ist ferner ein Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) an den erfindungsgemäßen Wirtszellen das Membranpotential mißt,
b) die Wirtszellen mit einer Substanz in Kontakt bringt und
c) an den Wirtszellen erneut das Membranpotential mißt,
wobei der Unterschied zwischen dem Membranpotential vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die einzusetzende Konzentration der Substanz ist, um eine Änderung des Membranpotentials zu erreichen.

Gegenstand der Erfindung ist ferner ein Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) in den erfindungsgemäßen Wirtszellen das Meinbranpotential und den Kaliumauswärtsstrom mißt,
b) die Wirtszellen mit einer Substanz in Kontakt bringt und
c) das Membranpotential und den Kaliumauswärtsstrom erneut mißt,
wobei die Unterschiede zwischen dem Membranpotential und dem Kaliumauswärtsstrom vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die einzusetzende Konzentration der Substanz ist, um eine Änderung des Membranpotentials sowie des Kaliumauswärtsstromes zu erreichen.

Gemäß einer besonders bevorzugten Ausführungsform wird in den oben genannten, erfindungsgemäßen Verfahren jeweils vor Schritt a) zunächst geprüft bzw. nachgewiesen, ob die zu testenden Substanzen an in Bakterien hergestellte bakterielle Kaliumkanäle binden und deren Aktivität modulieren können.

Dazu werden zunächst DNA Sequenzen, welche für bakterielle Kaliumkanalproteine kodieren, vorzugsweise das lct B Gen (Barstow et al., Nucleic Acids Res. **15**:1331 (1987), in Bakterien, vorzugsweise E. coli, eingeführt und exprimiert. Beim Exprimieren wird vorzugsweise ein aus mehreren Histidinen bestehendes Peptid an das Kaliumkanalprotein angehängt. Die zugrundeliegenden Verfahren sind dem Fachmann bekannt (Sambrook et al, a.a.O.). Anschließend werden die auf diese Weise hergestellten bakteriellen Kaliumkanalproteine gereinigt. Dies geschieht vorzugseise mittels Nickelchelatchromatographie, wobei die an das Protein angehängten Histidine an das Nickel binden (pET System-Handbuch, 6. Ausgabe, 1995, Novagen Bad-Soden).

Die gereinigten Kaliumkanalproteine werden anschließend in Lipiddoppelschichten eingebaut (A.M. Correa et al., Biophys. J. 54 (1988) 569-575; P. Müller Proc. Natl. Acad. Sci. USA 69 (1962) 3561-3566; P. Mueller et al., Nature 194 (1962) 979-980). Danach wird mittels patch-clamp-Methode (s.o.) die Aktivität der Kaliumkanäle sowie die Fähigkeit der zu testenden Substanzen bestimmt, die Aktivität der bakteriellen Kaliumkanäle zu modulieren.

Gegenstand der Erfindung ist ferner die Verwendung der in SEQ ID NO: 1 dargestellten Nukleotidseguenz zur Expression eines bakteriellen Kaliumkanals in eukaryotischen Zellen. Bezüglich der geeigneten Zellen werden die weiter oben im Abschnitt "Wirtszellen" genannten Ausführungsformen übernommen.

Ferner ist Gegenstand der Erfindung die Verwendung eines Nukleinsäuremoleküls, das für Homologe, Mutanten, Derivate oder Fragmente des in SEQ ID NO: 1 dargestellten Nukleinsäuremoleküls kodiert, zur Expression eines bakteriellen Kaliumkanals in eukaryotischen Zellen. Homologe, Mutanten, Derivate oder Fragmente der in SEQ ID NO: 1 dargestellten Nukleotidsequenz sind dadurch gekennzeichnet, daß sie eine Nukleotidsequenz aufweisen, die einen Sequenzabschnitt umfaßt, der eine Homologie von mindestens 40 %, vorzugsweise von mindestens 60 % und besonders bevorzugt von mindestens 80 % zu dem von Nukleotid 172 bis Nukleotid 312 in SEQ ID NO: 1 dargestellten Sequenzabschnitt aufweist. Bezüglich der geigneten Zellen werden die weiter oben im Abschnitt "Wirtszellen" genannten Ausführungsformen übernommen.

Im Rahmen der vorliegenden Erfindung wird mit den erfindungsgemäßen Verfahren bevorzugt die antibiotische Wirkung von Substanzen getestet. Erfindungsgemäß bedeutet antibiotisch, daß die Stoffe bakteriostatische und/oder bakteriozide Eigenschaften haben. Dadurch ist es möglich, Substanzen zu identifizieren, die sich als Antibiotika eignen.

Die Erfindung wird im folgenden durch Figuren, Beispiele und Sequenzprotokolle verdeutlicht.

### Kurze Beschreibung der Figuren

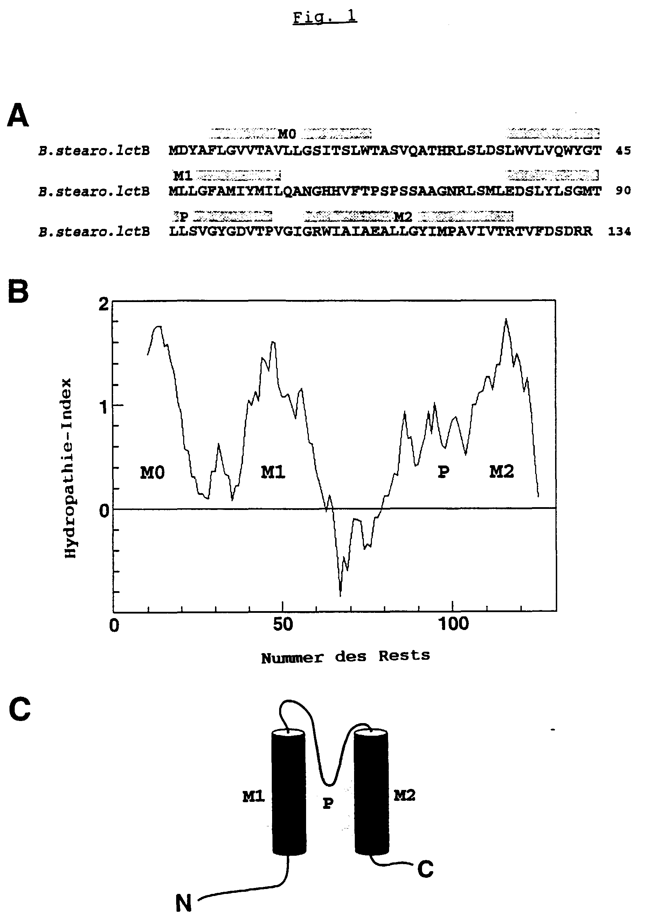

Figur 1: Sequenz und mögliche Membran-spannende Topologie der Bacillus Lct B Kaliumkanaluntereinheit. **A:** Abgeleiteter offener Leserahirten des *Bacillus stearothermophilus lct* B Gens. Aminosäuren sind im Einbuchstabencode abgekürzt. Die Numerierung an der rechten Seite bezieht sich auf die jeweils letzte Aminosäure. Die aus der Hydropathie-Analyse in **B** vorhergesagten hydrophoben Segmente sind mit grauen Balken markiert sowie die P-Region, die die charakteristische Kaliumkanalsignatursequenz enthält. **C:** Mögliche Topologie einer Membran-insertierten Lct B Kaliumkanaluntereinheit. N: Aminoterminus; C: Carboxyterminus; die hydrophoben Segmente M1 und M2 sind als schwarze Zylinder dargestellt, die die Membran, schattiert in Grau, durchspannen.

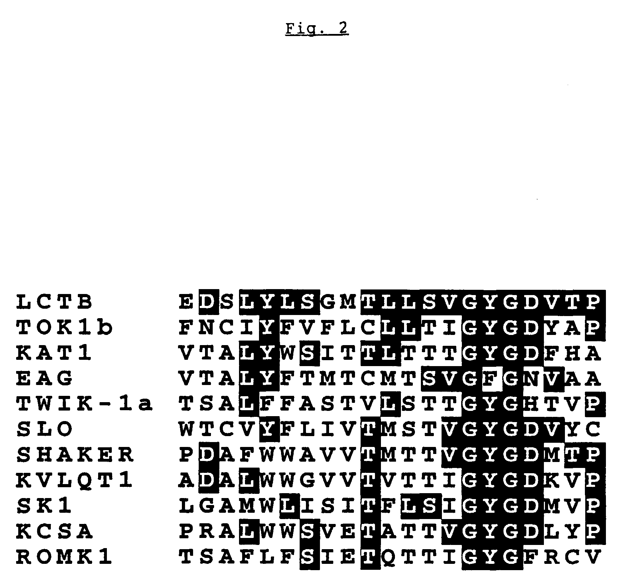

Figur 2: Sequenzvergleich der Aminosäuresequenzen (im Einbuchstabencode) der P-Regionen repräsentativer Mitglieder der Kaliumkanalproteinsuperfamilie. Aminosäuren, die zur Lct B-Sequenz identisch sind, sind schwarz unterlegt. Die Sequenzen sind unter folgenden Nummern in Gensequenzenbibliotheken (EMBL) zugänglich: EAG (Q02280), KAT1 (M86990), KCSA (Z37969), KVLQT1 (V40990), LCTB (X05067), ROMK1 (X72341), SHAKER (M17211), SK1 (U69885), SLO (M69053), TOK1b (P40310), TWIK-1a (U33632).

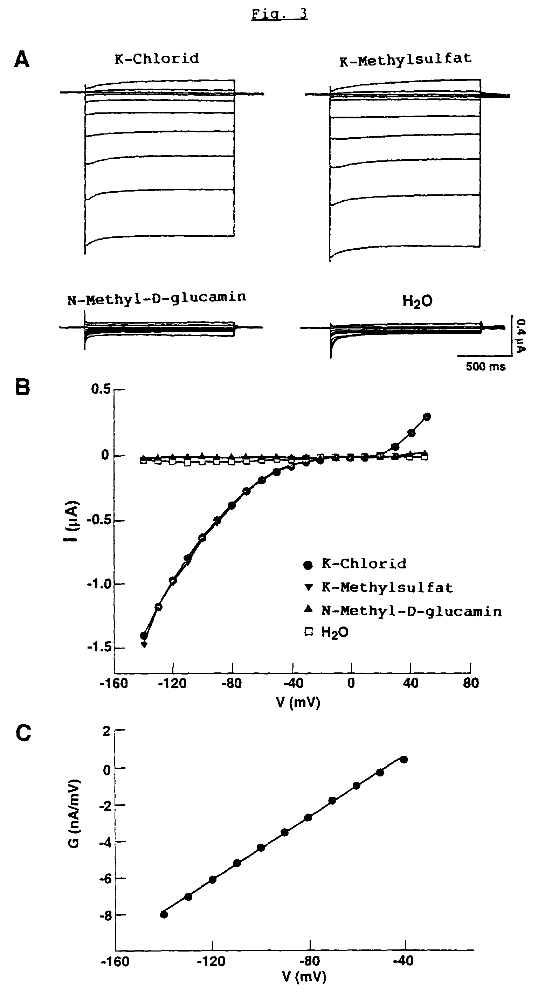

Figur 3: Lct B - Kaliumströme hervorgerufen in Xenopus Oozyten nach Injektion von Lct B RNA. Die Ströme wurden mit einer Zwei-Elektroden Spannungsklemme gemessen. Das Haltepotential war 0 mV, die Testpotentiale von 1.5 s Dauer reichten von -140 mV bis +50 mV in 10 mV Schritten. Zur besseren Übersicht werden nur Stromspuren für 20 mV Intervalle im Bereich von -140 mV bis +40 mV gezeigt. **A**: K-Chlorid: Standardlösung mit 90 mM KCl; K-Methylsulfonat: Chlorid in der Standardbadlösung wurde ersetzt durch Methylsulfat; N-Methyl-D: Glucamin-Kalium in der Standardbadlösung wurde durch N-Methyl-D-Glucamin ersetzt; H₂O-Kontrolle: Messungen, bei denen anstelle von *lct* B RNA H₂O in die Oozyten injiziert wurde. **B:** Strom (I) - Spannungs (V) Beziehungen für die in A gezeigten Ströme. Die Amplituden (Mittelwerte ± Standardabweichung, n=3) wurden 500 ms nach dem Anlegen der entsprechenden Testspannung gemessen.

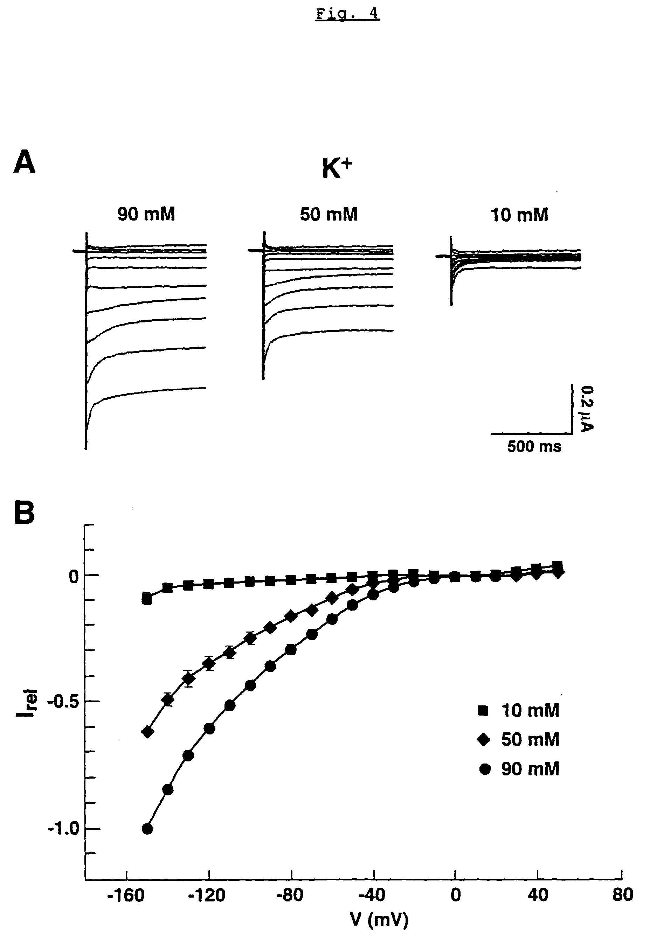

Figur 4: Lct B-Stromamplituden werden durch die Kaliumkonzentration in der Badlösung beeinflußt. **A:** Lct B-Ströme wurden wie in Abb. 3A gemessen. K⁺ in der Standardbadlösung (90 mM) wurde, wie angezeigt, reduziert durch Ersatz mit entsprechenden N-Methyl-D-Glucamin-Konzentrationen. Zur besseren Übersicht werden nur Stromkurven gezeigt für Testpuls-Intervalle von 20 mV im Spannungsbereich von -140 mV bis +40 mV. **B:** Strom-Spannungsbeziehungen für die in A gezeigten Ströme. Stromamplituden (Mittelwerte Standardabweichung, n=3) wurden 100 ms nach dem Anlegen der entsprechenden Testspannung gemessen. Iᵣₑₗ-Stromamplitude, normalisiert in Bezug auf die bei -120 mV gemessen Stromamplitude in der Standardbadlösung.

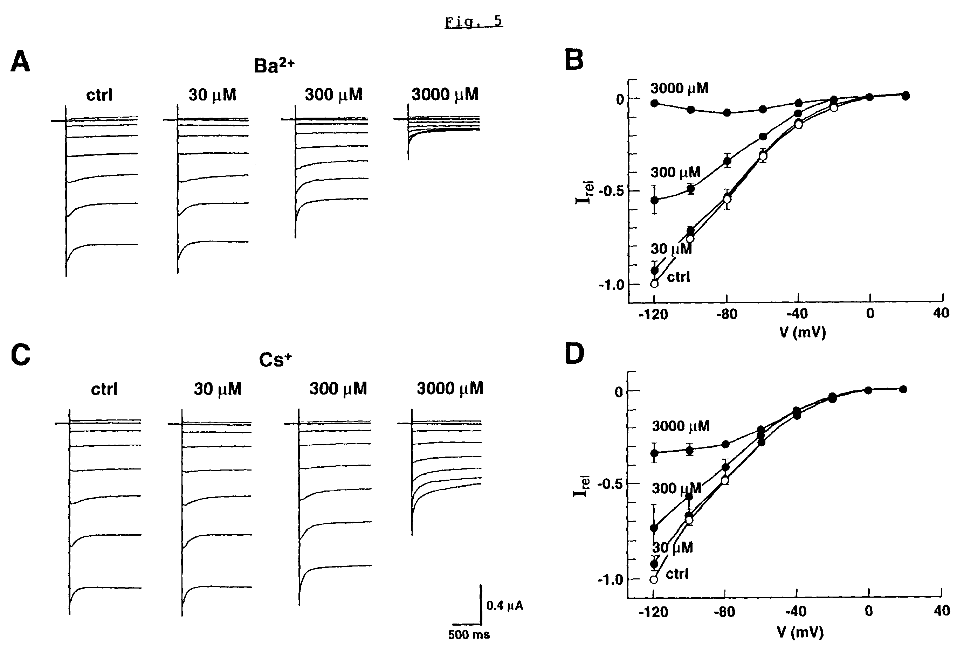

Figur 5: Inhibierung der Lct B-Ströme durch Ba²⁺ (A, B) und Cs⁺ (C, D). Ba²⁺ und Cs⁺ wurden zur Badlösung in den folgenden Konzentrationen hinzugegeben; 3, 30, 300, 3000 µM. Das Haltepotential war 0 mV; die Testpulse wurden im Bereich von -120 mV bis +20 mV in 20 mV-Schritten durchgeführt. **A,C:** Aktuelle Meßkurven; **B,D:** Graphische Auswertung der Mittelwerte (± Standardabweichungen) aus je vier Experimenten. Iᵣₑₗ-Stromamplitude, normalisiert in Bezug auf die bei -120 mV gemessene Stromamplitude in der Standardbadlösung.

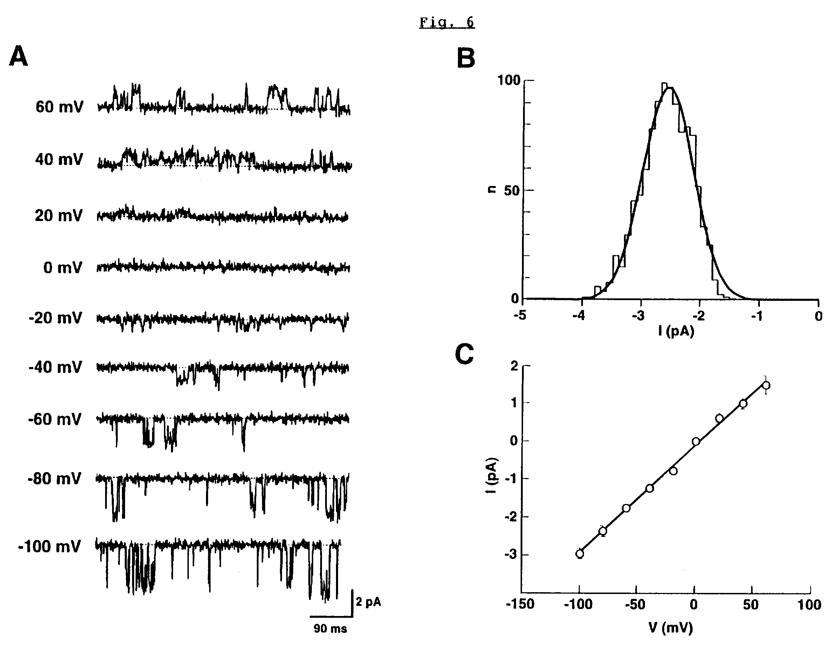

Figur 6: Lct B-Einzelkanalströme. **A**: Einzelkanalstromspuren. Das Haltepotential war 0 mV, die angelegten Testpotentiale sind auf der linken Seite angezeigt. **B:** Amplitudenhistogramm der gemessenen Einzelkanalströme bei +80 mV. Der angepaßte Mittelwert war bei -2.76 pA; n = Anzahl der Einzelkanalereignisse. **C:** Einzelkanalstrom-Spannungskurve. Die Datenpunkte entsprechen den Mittelwerten der aus 9 Patchen angepaßten Mittelwerte (sh. B). Die Fehlerbalken entsprechen der mittleren Standardabweichung. Die Steigung der Regressionsgeraden entspricht 28.2 pS.

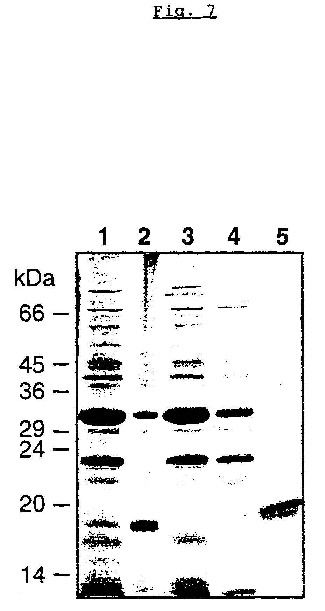

Figur 7: Reinigung des LctBHis-Proteins. Von jedem Aufarbeitungsschrift (s.a.o.) wurde ein Aliquot genommen und in einer SDS-Polyacrylamidgelelektrophorese untersucht. Die Färbung der Proteinbanden erfolgte mit Silbernitrat. Bahn 1: Überstand der Sucrosedichtegradientenzentrifugation; Bahn 2: Homogenisat des Sediments der Sucrosedichtegradientenzentrifugation; Bahn 3 und 4: Waschfraktionen der Nickelchelat-Säulenchromoatographie; Bahn 5: Elutionsfraktion der Nickelchelatsäule mit gereinigtem LctBHis-Protein.

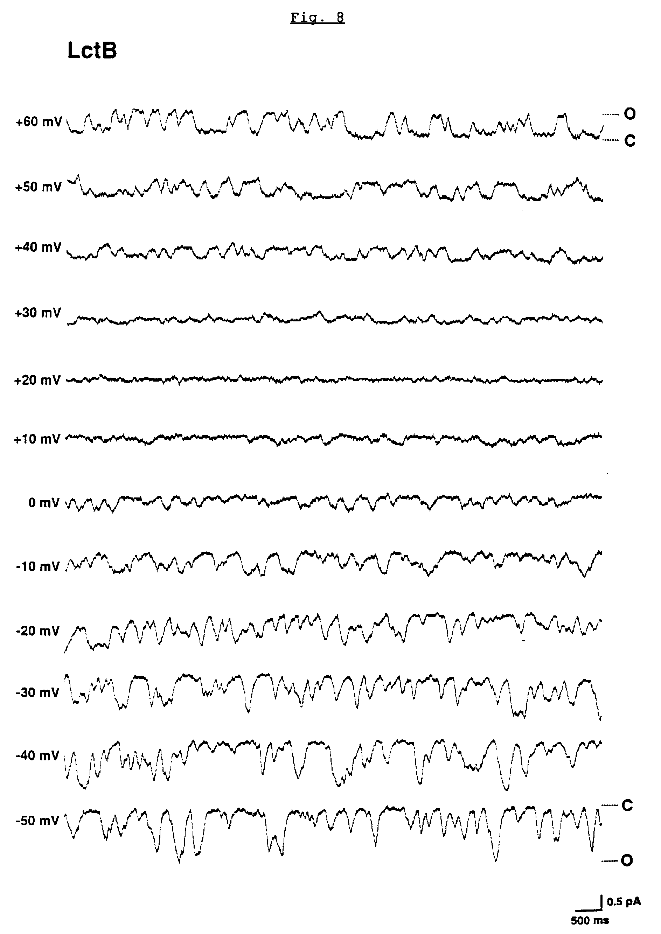

Figur 8: Zu sehen sind Kanalartige Leitfähigkeiten, die nach Zugabe von 50 µl LctBHis-Protein/Proteoliposomenlösung zu 450 mM KCl enthaltenden Seite einer Teflonküvette mit einer optisch schwarzen Doppellipidschicht aufgenommen wurden. Die Kanalaktivitätsspuren wurden bei den angegebenen Spannungen registriert. C - geschlossener Kanal; O - offene Kanäle. Zeitachse und Stromamplitude sind unten rechts angegeben.

### Beispiele

### Beispiel 1

### Herstellung von lct B RNA

Die Sequenz der *lct* B Gene aus *Bacillus caldotenax* und *Bacillus stearothermophilus* (*B.stearo*) wurde von Barstow et al. Nucleic Acids Research 15, 1331 (1987) veröffentlicht.

Die den offenen *lct* B Leserahmen enthaltende DNA Sequenz wurde im Rahmen der vorliegenden Erfindung in den Expressionsvektor pCDNA3 (Stratagene) einkloniert, so daß *in vitro* eine RNA synthesisiert werden konnte, die 5' zum ATG Startcodon eine Kozaksequenz enthält, die gecapped ist und am 3' Ende mit einem Poly A-Schwanz versehen ist. Dazu wurde das *lct* B Gen in einer Polymerasekettenreaktion amplifiziert mit *Bacillus stearothermophilus* DNA (Deutsche Sammlung von Mikroorganismen und zellkulturen GmbH, Braunschweid, Katalog 1996 - Nr. 6834) als Template und den Primern 5'-ATTGGATCCGCCGCCACCATGGATTACGCATTCCTC-(Sense, SEQ ID NO:8) und 5'-CTCTCTAGATCAACGGCGATCCGAG- (Antisense, SEQ ID NO:8). Der Sense-Primer enthält eine Kozak-Konsensussequenz und eine Schnittstelle für das Restriktionsenzym Bam HI. Der Antisense-Primer enthält eine Schnittstelle für das Restriktionsenzym Xba I. Zur Amplifikation in 50µl Gesamtvolumen wurden 1 ng *B. stearo* DNA, 50 pM sense-Primer, 50 pH antisense-Primer, je 200 µM dNTP, 10 µl 100mM Tris-HCl (pH8.4), 250mM KCl, 6.5mM MgCl₂ und 2.5 Einheiten Taq-DNA-Polymerase folgendermaßen inkubiert: 3 min 95 °C, gefolgt von 20 Zyklen 1 min 52 °C, 1,5 min 72 °C, 1 min 94 °C. Nach Beendigung der Zyklen wurde der Ansatz 1 min auf 94 °C geheizt und anschließend 10 min bei 72 °C inkubiert und auf 4°C abgekühlt. Das PCR-Produkt wurde durch Agarose-Gelelektrophorese nach Standardmethoden gereinigt (Sambrook, J., Fritsch, E.F. and Maniatis, T. (1989) Molecular Cloning: A laboratory Manual, 2^{nd}ed; Cold Spring Harbor Laboratory Press. New York.).

Das amplifizierte PCR-Produkt wurde mit Bam HI und Xba I (jeweils Boehringer Mannheim, Verdau wurde entsprechend den Angaben des Enzymherstellers durchgeführt) verdaut. Das entstandene Bam HI/Xba I DNA-Fragment wurde in den Bam HI/Xba I-verdauten Vektor pGEMHE (Stratagene) kloniert (Sambrook, J., Fritsch, E.F. and Maniatis, T. (1989) Molecular Cloning: A laboratory Manual, 2^{nd}ed; Cold Spring Harbor Laboratory Press. New York.). Die Integrität des Konstrukts wurde durch Sequenzierung der pGEMHE-*lct* B-Plamid DNA überprüft (Sanger, F., Nicklen, S. and Coulson, A.R. (1977) DNA sequencing with chainterminating inhibitors. Proc. Natl. Acad. Sci. USA 74, 5463-5467). Für die *in vitro* Synthese von *lct* B RNA wurde pGEMHE-*lct* B-Plamsid DNA mit PstI linearisiert (Sambrook, J., Fritsch, E.F. and Maniatis, T. (1989) Molecular Cloning: A laboratory Manual, 2^{nd}ed; Cold Spring Harbor Laboratory Press. New York.). Die linearisierte DNA diente als Template für T7 RNA-Polymerase. Die Synthese gecappter RNA erfolgte unter Verwendung des Ambion Mmessage mMachine® *in vitro* Transkriptions Kits (Ambion; der Kit umfaß tim wesentlichen T7 Polymerase, rNTPs einschließlich Cap-Analoga und DNAse I). Die *lct* B RNA wurde mit Ethanol präzipitiert (Sambrook, J., Fritsch, E.F. and Maniatis, T. (1989) Molecular Cloning: A laboratory Manual, 2^{nd}ed; Cold Spring Harbor Laboratory Press. New York.) und in H₂O zu einer Konzentration von 0.5 mg/ml aufgenommen.

### Beispiel 2

### Expression des lct B-Gens in Xenopus Oozyten

Xenopus Oozyten des Stadiums V oder VI wurden aus anästhetisierten Fröschen manuell präpariert (Miledi, R. and Woodward, R.M. (1989) Effects of defolliculation on membrane current responses of Xenopus oocytes. J. Physiol. 416, 601-621). Die Anästhesie erfolgte durch 10 minütige Behandlung mit 5 mM 3-Aminobenzoesäureäthylester (Sigma). Die Oozyten wurden bei 20 °C in einem Medium inkubiert, das 88 mM NaCl, 1mM KCl, 1.5 mM CaCl₂, 2.4 mM NaHCO₃, 0.8 mM MgSO₄, 5 mM HEPES (pH 7.4) und 100 ng/ml Penicillin und 100 µg/ml Streptomycin enthielt. 3-8 µg RNA (gelöst in 50 µl H₂O) wurden in jede Oozyte injiziert. Drei bis fünf Tage später wurden die *Xenopus* Oozyten elektrophysiologisch untersucht, dabei wurde einen Tag vor den elektrophysiologischen Experimenten das follikuläre Gewebe manuell von den Oozyten entfernt.

### Beispiel 3

### Elektrophysiologische Untersuchung der injizierten Oozyten

Die *Xenopus* Oozyten wurden drei bis fünf Tage nach der Injektion der RNA mit einer konventionellen Zweielektroden-Spannungsklemme elektrophysiologisch untersucht. Die Ausgangssspannungsklemme betrug bei den Messungen 0 mV. Spannungspulse von 1.5 s Dauer wurden im Bereich von +50 mV bis -150 mV angelegt (Figur 3). Die verwendeten Mikroelektroden waren aus Borosilikatglass und hatten einen Widerstand zwischen 0.4 bis 2 MΩ. Die Badlösung bei den Messungen enthielt 90 mM KCl, 3 mM MgCl₂, 5 mM HEPES (pH 7.4) und 300 mM Niflumsäure. Die Niflumsäure wurde eingesetzt, um mögliche Oozyten-endogene Chloridströme, die bei den Messungen stören wurden, zu unterdrücken. Bei einigen Messungen enthielt die Badlösung niedrigere KCl-Konzentrationen als 90 mM. Die entsprechende Differenz wurde durch äquimolaren Ersatz mit N-Methyl-D-Glucamin ausgeglichen. Ba²⁺ und Cs⁺ wurden bei Bedarf der Badlösung in Konzentrationen von 3, 30, 300 bzw. 3000 nmol/l zugesetzt. Einzelkanalströme wurden auf 0 mv geklemmt, bevor hyperpolarisierende Spannungspulse im Bereich bis zu -100 mV angelegt wurden. Da das Membranpotential der Oozyten in Kontrollösungen zwischen -6 und -13 mV (n=5) schwankte, wurde das Pipettenpotential korrigiert, um ein angenommenes intrazelluläres Potential von -10mV einzustellen. Die Patchpipetten wurden aus Borosilikatglass gezogen und hatten an der Spitze einen Durchmesser von ca. 2 µm. Die Widerstände lagen zwischen 2 und 3 MΩ. Alle Messungen wurden bei einer Temperatur von 22 ± 1 °C durchgeführt.

Die verwendete Meßapparatur war eine Standard-Meßapparatur und enthielt als Verstärker einen EPC-7 Verstärker (Listelectronic). Leckströme wurden von den Meßdaten abgezogen. Die Leckströme wurden anhand von Testpulsdaten, die bei +10 mV erhalten wurden, abgeschätzt. Dies geschah unter der Annahme, daß sie sich linear mit den angelegten Spannungen ändern. Einzelkanalströme wurden bei 2 kHz mit einem Niedrigbandfilter von 1 kHz aufgenommen. Zur Bestimmung von Einzelkanalamplituden bei 0 mV wurde mit 0.1 kHz gefiltert. Die Datenanalyse erfolgte mit der pCLAMP software (Axon Instruments).

Depolarisation der Oozytenmembran zu negativen Potentialen rief einen Einwärtsstrom hervor, der im Millisekundenbereich schnell zu einem Plateauwertanstieg und im Verlauf der Testpulse kaum abfiel. Sehr kleine oder gar keine Ströme wurden zwischen Testpotentialen von -30 mV und +10 mV gemessen, und Auswärtsströme wurden bei Testpotentialen gemessen, die positiver als 20 mV waren (Figur 3A). Die Auswärtsströme stiegen langsamer zu einem Plateauwert an als die Einwärtsströme. Identische Ströme wurde erhalten, wenn Chlorid als Anion in der Badlösung durch Methylsulfat ausgetauscht wurde (Figur 3A). Im Gegensatz dazu wurden keine meßbaren Ströme erhalten, wenn die Kaliumionen in der Badlösung durch N-Methyl-D-Glucamin ausgetauscht wurden. Ebenfalls führte in Kontrollmessungen die Injektion von H₂O nicht zu Strömen, die mit denen von den Lct B-Poteinen hervorgerufenen vergleichbar waren. Diese Ergebnisse zeigen, daß das *lct* B-Gen für Untereinheiten eines Kalium-selektiven Ionenkanals kodierte.

Die Stromkurven in Figur 3A wurden ausgewertet, indem die maximalen gemessenen Stromamplituden bei der jeweiligen Spannung gegen die Spannung des entsprechenden Testpulses (I-V-Kurve) aufgetragen wurden (Figur 3B). Die I-V-Kurven für die *lct* B-vermittelten Ströme zeigen eine "doppelte Rektifizierung", d.h. bei Testpotentialen negativer als -40 mV ist eine Abwärtsbiegung zu beobachten und bei Testpotentialen positiver als +30 mV eine Autwärtsbiegung. Dies zeigt für die *lct* B-vermittelten Ströme sowohl eine biphasische wie eine starke Spannungsabhängigkeit.

Kalium-selektive Kanäle leiten Kalium in der Regel entsprechend dem vorliegenden elektrochemischen Gradienten, der durch die Nernst-Gleichung berechnet werden kann. Dementsprechend veränderten sich die Lct B-Stromamplituden und Schwellenpotentiale für die Lct B Ströme bei Absenkung der externen Kaliumkonzentration, die den vorhersagbaren, berechneten Kaliumgleichgewichtspotentialen E_{K} entsprachen (Figur 4). Wenn die Kaliumionenkonzentration in der Badlösung 50 mM betrug, lag das Schwellenpotential, definiert als das Potential, bei dem der Einwärtsstrom mehr als 1% des bei -150 mV gemessenen maximalen Stroms beträgt, bei ca. -20 mV. Wenn die Kaliumionenkonzentration der Badlösung 10 mM betrug, lag das Schwellenpotential bei ca. -58 mV. Die Lct B-Kanäle sind durch externe Ionen wie Ba²⁺ und Cs⁺ blockierbar (Figur 5A). Die Blockierung der Lct B-Kanäle durch Ba²⁺ der Lct B-Kanäle ist spannungsabhängig (Figur 5B), so daß die Sensitivität der Kaliumkanäle gegenüber Ba²⁺ bei negativen Potentialen zunimmt. Die Konzentration, die notwendig war, um 50 % der Stromamplitude zu blockieren (IC₅₀) beträgt bei -120 mV 0.33 mM. Im Falle von Cs⁺ (Figur 3A,B) beträgt der IC₅₀ bei -120 mV 1.1 mM.

Im Gegensatz zu den I-V-Kurven, die mit der Zweielektrodenklemme gemessen wurden, sind die Einzelkanal I-V-Kurven nicht biphasisch, sondern linear (Figur 6). Daraus ergibt sich, daß die Einzelkanalleitfähigkeit des offenen Kanals wahrscheinlich spannungsunabhängig ist, während die Offenwahrscheinlichkeit des Kanals deutlich spannungsabhängig ist.

Die Einzelkanalmessungen wurden in der dem Fachmann bekannten "cell-attached" Konfiguration (Hille, B. (1992) Ionic channels of excitable membranes. Sunderland, MA: Sinauer. 2^{nd}ed.) im Testpotentialbereich von -100 mV bis +60 mV durchgeführt. Es wurden ferner die gemittelten Einzelkanalamplituden gegen die Spannung aufgetragen. Unter den oben genannten Meßbedingungen wurde für die Lct B-Kanäle eine mittlere Leitfähigkeit von 27.8 ± 0.9 pS bestimmt (Figur 6C).

Die funktionellen Eigenschaften des Lct B-Kanals zeigen, daß keine direkte funktionelle Verwandtschaft zu bisher klonierten und exprimierten eukaryotischen Kaliumkanälen aufzeigten. Aufgrund dieser Daten und der geringen Sequenzidentität (<40% Homologie zu anderen Kaliumkanälen) handelt es sich bei den Lct B Proteinen um eine neue, eigenständige Kaliumkanalfamilie.

### Beispiel 4:

### Herstellung und Reinigung von LctB-Kanälen in bakteriellen Expressionssystemen.

Um das LctB Protein in E. coli herstellen zu können, wurde der offene LctB Leserahmen in den Translationsvektor pET25b (Novagen, Schwalbach, Deutschland) kloniert. Dazu wurde das lctB-Gen aus genomischer DNA von *Bacilius stearothermophilus* mittels PCR (Bedingungen s. unten Beispiel 3) unter Verwendung der Primerpaare LctB Nde 1625 (5'-CAGAGGTACATATGGATTACGCATTCCTCGGA-3', SEQ ID NO:6) und LctB Bam25 (5'-AAGGATCCGAACGGCGATCCGAGTCAAATA-3', SEQ ID NO:7) amplifiziert. Das amplifizierte Fragment wurde über die in den Primern bzw. dem Vektor vorhandenen NdeI- und BamHI-Schnittstellen in den pET25b Vektor kloniert. Es wurde das Expressionsplamid pLctbHis erhalten. Zur Expression des mit einer Polyhistidinsequenz am C-Terminus versehenen LctB-Proteinsequenz wurden *E.coli* Bakterien des Stammes BL21(DE3) (Stratagene, Heidelberg, Deutschland) mit pLctbHis-DNA transformiert, induziert und bei 37°C kultuviert. Diese Verfahren sind dem Fachmann gut bekannt. Nach 4 Stunden wurde die Inkubation abgebrochen. Die Bakterien wurden abzentrifugiert in einer Beckmann (München, Deutschland)-Zentrifuge (Typ J2-21, 7000 UpM, 100 min., 4°C). Das Bakteriensediment wurde in 10 mM Tris/HCl (pH 8.0), 1 mM EDTA, 2 mM DTT (7 ml Puffer pro 100 ml Bakterienkultur) aufgenommen und mit Lysozym (Sigma, Steinheim, Deutschland) (0.1 mg/ml, 100 min., 30°C) versetzt. Anschließend wurden in einem 100 ml-Röhrchen in einer Parr-Bombe (Parr Instrument Co., Illinois, USA) die Bakterien mit Druck behandelt. Die Druckbehandlung erfolgte 15 min. mit 900 psi bei 4°C und unter Stickstoff. Am Auslaufstutzen lysierten die Bakterien durch einen plötzlichen Druckausgleich und wurden in einem 10 ml-Röhrchen aufgefangen. Die erhaltenen Spheroblasten wurden mit 1 Volumen 100 mM HEPES (pH 7.5), 100 mM EDTA versetzt. Noch vorhandene Bakterienzellen wurden durch Zentrifugation in einer Sigma (Steinheim, Deutschland) 2K Zentrifuge (8000 UpM, 100 min., 4°C) entfernt. Der überstand (6 ml) wurde über ein Sucrosekissen geschichtet, das aus 32 ml 10 mM HEPES (pH 7.5), 10 mM EDTA, 40% Sucrose (w/v) bestand und zentrifugiert (Beckmann (München, Deutschland) Ultrazentrifuge L2-65B, Rotor SW 28, 25000 UpM, 18 h, 4°). Nach der Zentrifugation wurde das Sediment, das die äußeren Membranfraktionen enthielt mit 10 mM HEPES (pH 7.5), 400 mM NaCl, 100 mM KCl, 5 mM Imidazol-Puffer (20 ml pro 12 Bakterienausgangskultur) versetzt und in einem Dounce-Homogenisator mit einem Teflonpistill homogenisiert. Das Homogenat wurde mit 1 Volumen 10 mM HEPES (pH 7.5), 400 mM NaCl, 100 mM KCl, 5 mM Imidazol, 20 mM Mega 9-Puffer versetzt und 1 h bei Raumtemperatur inkubiert. Nach Ultrazentrifugation (Beckmann (München, Deutschland) Ultrazentrifuge L5-65B, Kontron Rotor KA-30-50, 100000 g, 1 h, 20°C) wurde das solubilisierte Protein dekantiert und auf eine Nickelchelatsäule (His Bind metal chelation resin, Novagen (Schwalbach, Deutschland); 2.5ml Säulenvolumen) aufgetragen. Die Säule war vorher mit 6 Säulenvolumen einer 50 mM NiSO₄-Lösung beladen und mit 4 Säulenvolumen 10 mM HEPES (pH 7.5), 400 mM NaCl, 100 mM KCl, 5 mM Imidazol, 20 mM Mega-9 Puffer äquilibriert worden. Die Säule wurde mit 6 Säulenvolumen 10 mM HEPES (pH 7.5), 400 mM NaCl, 100 mM KCl, 20 mM Imidazol, 20 mM Mega-9 Puffer und mit 4 Säulenvolumen 10 mM HEPES (pH 7.5), 400 mM MaCl 100 mM KCl, 200 mM Imidazol, 20 mM Mega-9 Puffer gewaschen. Die Elution des LctBHis-Proteins (vgl. SEQ ID NO: 5) erfolgte mit zwei Säulenvolumen 10 mM HEPES (pH 7.5), 400 mM NaCl, 100 mM KCl, 500 mM Imidazol, 20 mM Mega-9 Puffer. Die Reinheit des isolierten LctBHis-Proteins wurde mittels einer denaturierenden Polyacrylamidgelelektrophorese (Laemmli, U.K (1970) Nature 227, 680-685) überprüft (s. Figur 7).

Die Eletrophorese wurde in einer Mini-Protean II Eletrophoresezelle (BioRad, München, Deutschland) durchgeführt. Das Trenngel (Höhe 4.5 cm, Dicke 0.75 mm) wurde mit einer 10% (v/v) Acrylamidlösung (National Diagnostics (Hessisch-Oldendorf, Deutschand), Protogel; Acrylamid zu Bisacrylamidverhältnis 30:0.8) in 1x Trenngelpuffer mit 1 mg/ml Ammoniumpersulfat und 1 µl/ml N,N,N*',*N*'*-Tetramethyldiamin (TEMED) hergestellt. Polymerisationsdauer bei Raumtemperatur war 1 h. Der Trenngelpuffer war 0.4 M Tris/HCl (pH 8.8) und 0.1% (w/v) Natriumdodecylsufat (SDS). Das Trenngel wurde mit einem Sammelgel überschichtet (Höhe 0.8 cm, Dicke 0.75 mm), das aus 3% (w/v) Acrylamidlösung in 120 mM Tris/HCl (pH 6.8), 0.1% (w/v) SDS Puffer mit 1 mg/ml Ammoniumpersulfat und 1 µl/ml TEMED bestand. Die Proteinproben (20 µl) wurden mit 1 Volumen 250 mM Tris/HCl (pH 6.8), 5% (w/v) SDS, 10% (w/v) β-Mercaptoäthanol, 20% (w/v) Glyzerin, 0.05% (w/v) Bromphenolblau versetzt und 10 min. auf 95% erhitzt und auf das Gel aufgetragen. Die Elektrophorese wurde bei einer Spannung von 200 Volt in 2.5 mM Tris, 25.5 mM Glycin, 0.1% (w/v) SDS-Puffer durchgeführt. Der Lauf wurde beendet, wenn das Bromphenolblau in der auf getragenen Probe die Unterkante des Trenngels erreicht hatte. Danach wurde das Polyacrylamidgel gewässert und in eine passende Glaswanne transferiert und mit einer Silbernitratlösung angefärbt (0.5 g Natriumcitrat, 200 mg Eisen (II) Sulfat und 50 mg Silbernitrat in 25 ml H₂0). Die Inkubation erfolgte für 3 min. und wurde durch mehrfaches Waschen in H₂O beendet, bis die Proteinbanden deutlich sichtbar wurden.

### Beispiel 5:

### Aktivitätstest des gereinigten LctBHis-Proteins nach Rekonstitution des LctB-Kanals in Lipiddoppelschichten.

Es wurden nach dem Verfahren von Montal und Mueller (Montae, M. u. Mueller, P. (1972) *Proc. Natl. Acad. Sci. USA* **69**, 3561-3566) Lipiddoppelschichten in einer Küvette hergestellt. Der gereinigte LctB-Kanal wurde in solubilisiertem Zustand in die Elektrolytlösung auf einer Seite der Membran gegeben. Hierzu war das gereinigte LctB-Protein von Detergens befreit. 1.5 ml des gereinigten LctBHis-Proteins wurde mit 1.5 ml Lipidlösung versetzt. Die Lipidlösung bestand aus Diphytanoyl phosphatidylcholin (DPPC), 1-Palmitoyl-2-oleyl-phosphatidyläthanolamin (POPE), 1-Palmitoyl-2-oleyl-phospatidylserin (POPS), Verhältnis 7:3:1 (w/w/w) in H₂O. Zur Liposomenbildung wurde die Lösung Ultraschall-behandelt (Branson Sonifier 250, Stufe 2, 70% gepulst, 4x1 min.).

Die LctBHis-Protein/Proteoliposomenlösung wurde mit 1/3 Volumen BioBeads SM2 (BioRad, München, Deutschland) versetzt, mit Stickstoff überschichtet und über Nacht bei 4°C langsam gerührt. Die BioBeads wurden durch frische BioBeads ausgetauscht und die Lösung wurde erneut gerührt (1 h, Raumtemperatur, unter Stickstoff). Die Proteoliposomenlösung wurde zentrifugiert (13000 UpM, 5 min, Sigma (Steinheim, Deutschland) 2K Zentrifuge). Der Überstand wurde fünfmal dialysiert gegen 10 mM HEPES (pH 7.0) 150 mM KCl, 1 mM CaCl₂-Puffer dialysiert (Dialyseschlauch von Spectra/Por, Spektrum MWCO 15000; 5x3 h gegen jeweils 300 ml Puffer bei Raumtemperatur).

Die Küvette bestand aus Teflon mit zwei Kompartimenten, die durch ein Loch von 1 mm² Fläche in der Trennwand verbunden waren. Beide Kompartimente waren mit jeweils 1.3 ml einer 450 mM bzw. 50 mM KCl-Lösung gefüllt und mit einem kleinen Rührmagneten bestückt.

5 µl Membranlipidlösung (1% (w/v) POPS/POPE im Verhältnis 1:3 in n-Dekan) wurden zur Herstellung einer optische schwarzen Lipiddoppelschicht über eine Seite der Apertur pipettiert. Beide Kompartimente der Küvette wurden über Agar/Agar-Salzbrücken (3 M KCl) und Ag/AgCl-Elektroden nach dem Fachmann gut bekannten Methoden mit einem Strom/Spannungs-Wandler verbunden. Das Stromsignal wurde mit 5-20 HZ gefiltert und verstärkt. Die der Lipiddoppelschicht abgewandte Seite war geerdet. Die Meßdaten wurden mit dem Programm pCLAMP 6 (Axon Instrument (Hamburg, Deutschland), Inc.) ausgewertet.

Das gereinigte LctB-Kanalprotein wurde als Proteoliposomenlösung (s.o., 25-199 µl) zum Proteoliposomenlösung (s.o., 25-100 µl) zum Einbau in die Lipid-Doppelschicht auf der Seite der Küvette zugegeben, die 450 mM KCl enthielt. Der Einbauprozess war nach ca. 45 Minuten abgeschlossen. LctB-Kanalaktivität wurde gemessen, indem die Spannung auf verschiedenen Haltepotentiale von -100 mV bis +100 mV in 10 mV Inkrementen geklemmt wurde (s. Figur 8).

Im Rahmen der Herstellung von gereinigten LctB-Kanälen in Liposomen ist auch denkbar, die Aktivität des Kanals mittels Rubidiumfluxmessungen (s.a.o.) durchzuführen.

## Patentansprüche

1. Eukaryontischer Expressionsvektor, der mindestens eine Nukleotidsequenz, die für eine Kaliumkanaluntereinheit kodiert, in einer Anordnung enthält, die die funktionelle Expression in Eukaryonten ermöglicht, **dadurch gekennzeichnet,** daß die Nukleotidsequenz die in SEQ ID NO: 1 dargestellte Sequenz umfaßt oder ein Homologes, eine Mutante, ein Derivat oder ein Fragment derselben ist.

2. Eukaryontischer Expressionsvektor nach Anspruch 1, **dadurch gekennzeichnet,** daß die Nukleotidsequenz bakteriellen Ursprungs ist.

3. Eukaryontischer Expressionsvektor nach Anspruch 1 oder 2, **dadurch gekennzeichnet,** daß das Homologe eine Nukleotidsequenz aufweist, die einen Sequenzabschnitt umfaßt, der eine Homologie von mindestens 40 % zu dem von Nukleotid 172 bis Nukleotid 312 in SEQ ID NO: 1 dargestellten Sequenzabschnitt aufweist.

4. Eukaryontischer Expressionsvektor nach Anspruch 1 oder 2, **dadurch gekennzeichnet,** daß das Homologe eine Nukleotidsequenz aufweist, die einen Sequenzabschnitt umfaßt, der eine Homologie von mindestens 60 %, vorzugsweise von mindestens 80 %, zu dem von Nukleotid 172 bis Nukleotid 312 in SEQ ID NO: 1 dargestellten Sequenzabschnitt aufweist.

5. Nukleinsäuremolekül, das für eine Kaliumkanaluntereinheit kodiert, **dadurch gekennzeichnet,** daß es einen Sequenzabschnitt umfaßt, der eine Homologie von mindestens 40 % zu dem von Nukleotid 172 bis Nukleotid 312 in SEQ ID NO: 1 dargestellten Sequenzabschnitt aufweist.

6. Nukleinsäuremolekül, das für eine Kaliumkanaluntereinheit kodiert, **dadurch gekennzeichnet,** daß es einen Sequenzabschnitt umfaßt, der eine Homologie von mindestens 60 %, vorzugsweise von mindestens 80 %, zu dem von Nukleotid 172 bis Nukleotid 312 in SEQ ID NO: 1 dargestellten Sequenzabschnitt aufweist.

7. Nukleinsäuremolekül nach den Ansprüchen 5 oder 6, **dadurch gekennzeichnet,** daß es für eine bakterielle Kaliumkanaluntereinheit kodiert.

8. Nukleinsäuremolekül nach den Ansprüchen 5 bis 7, **dadurch gekennzeichnet,** daß es eine RNA ist.

9. RNA-Molekül, **dadurch gekennzeichnet,** daß es die Nukleotide 109 bis 510 aus SEQ ID NO: 3 oder ein Nukleinsäuremolekül nach Anspruch 8 zusammen mit für die Translation wichtigen Regulationssequenzen umfaßt.

10. RNA-Molekül nach Anspruch 9, **dadurch gekennzeichnet,** daß es die in SEQ ID NO:3 gezeigte Nukleotidsequenz umfaßt.

11. Eukaryontische Wirtszelle, **dadurch gekennzeichnet,** daß sie mit einem Expressionsvektor nach den Ansprüchen 1 bis 4 transformiert ist.

12. Eukaryontische Wirtszelle, **dadurch gekennzeichnet,** daß sie mit einem RNA-Molekül nach den Ansprüchen 9 oder 10 transfiziert ist.

13. Eukaryontische Wirtszelle nach den Ansprüchen 11 oder 12, **dadurch gekennzeichnet,** daß sie eine CHO Zelle ist.

14. Eukaryontische Wirtszelle nach den Ansprüchen 11 oder 12, **dadurch gekennzeichnet,** daß sie ein Xenopus Oozyt ist.

15. Eukaryontische Wirtszelle nach den Ansprüchen 11 bis 14, **dadurch gekennzeichnet,** daß sie einen funktionellen bakteriellen Kaliumkanal exprimiert.

16. Eukaryontische Wirtszelle nach Anspruch 11, **dadurch gekennzeichnet,** daß sie den bakteriellen Kaliumkanal auf ihrer Oberfläche exprimiert.

17. Eukaryontische Wirtszelle nach Anspruch 12, **dadurch gekennzeichnet,** daß sie ein Xenopus Qozyt ist, der mit einem RNA-Molekül nach Anspruch 10 oder mit einem Vektor nach Anspruch 1 transfiziert ist, wobei die Nukleotidsequenz die in SEQ ID NO: 1 dargestellte Sequenz umfaßt, und wobei sie den Kaliumkanal, der ein einwärtsgleichrichtender Kaliumkanal ist, exprimiert.

18. Baktenieller Kaliumkanal, erhältlich durch Expression des Nukleinsäuremoleküls mit der in SEQ ID NO: 1 dargestellten Nukleotidsequenz oder durch Expression der Nukleinsäuremoleküle nach den Ansprüchen 5 bis 7 oder durch Expression der RNA nach den Ansprüchen 9 oder 10.

19. Verfahren zur Expression eines bakteriellen Kaliumkanals in Eukaryontenmembranen, **dadurch gekennzeichnet,** daß man Wirtszellen nach den Ansprüchen 11 bis 17 unter geeigneten Bedingungen kultiviert, die die Expression des Kaliumkanals ermöglichen.

20. Verfahren nach Anspruch 19, **dadurch gekennzeichnet,** daß man Wirtszellen nach Anspruch 14 verwendet, wobei die Wirtszellen den Kaliumkanal, der ein einwärtsgleichrichtender Kaliumkanal ist, auf ihrer Oberfläche exprimieren.

21. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet,** daß man
a) an Wirtszellen nach den Ansprüchen 11 bis 17 den Kaliumauswärtstrom mißt,
b) die Wirtszellen mit einer Substanz in Kontakt bringt und
c) an Wirtszellen erneut den Kaliumauswärtsstrom mißt,
wobei der Unterschied zwischen dem Kaliumauswärtsstrom vor und nach Zugabe der Substanz die Aktivität der Substanz bestimmt.

22. Verfahren nach Anspruch 21, **dadurch gekennzeichnet,** daß man den Kaliumauswärtsstrom mit Hilfe der 'patch-clamp'-Methode bestimmt.

23. Verfahren nach den Ansprüchen 21 oder 22, **dadurch gekennzeichnet,** daß eine Substanz eine öffnende Substanz ist, wenn nach Zugabe der Substanz bei einem Membranpotential, bei dem ohne Zugabe der Substanz keine Kaliumauswärtsströme fließen, Kaliumauswärtsstöme fließen.

24. Verfahren nach den Ansprüchen 21 oder 22, **dadurch gekennzeichnet,** daß eine Substanz eine aktivierende Substanz ist, wenn nach Zugabe der Substanz ein bereits vorhandener Kaliumauswärtsstrom verstärkt wird.

25. Verfahren nach den Ansprüchen 21 oder 22, **dadurch gekennzeichnet,** daß eine Substanz eine schließende Substanz ist, wenn nach Zugabe der Substanz bei einem Meinbranpotential, bei dem ohne Zugabe der Substanz Kaliumauswärtsströme fließen, keine Kaliumauswärtsströme fließen.

26. Verfahren nach den Ansprüchen 21 oder 22, **dadurch gekennzeichnet,** daß eine Substanz eine inaktivierende Substanz ist, wenn nach Zugabe der Substanz ein bereits vorhandener Kaliumauswärtsstrom vermindert wird, ohne daß der Kaliumasuwärtsstrom vollständig zum Erliegen kommt.

27. Verfahren nach den Ansprüchen 21 oder 22, **dadurch gekennzeichnet,** daß eine Substanz eine verändernde Substanz ist, wenn durch Zugabe der Substanz biophysikalische Eigenschaften des Kaliumkanals wie Spannungsabhängigkeit, Leitfähigkeit, Aktivierungszeitkonstanten, Inaktivierungszeitkonstanten, Schaltverhalten, Offenzeiten oder Geschlossenzeiten verändert werden.

28. Verfahren nach den Ansprüchen 21 oder 22, **dadurch gekennzeichnet,** daß eine Substanz eine verändernde Substanze ist, wenn durch Zugabe der Substanz die Zelloberflächenexpression des Kaliumkanals verändert wird.

29. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet,** daß man
a) an Wirtszellen nach den Ansprüchen 11 bis 17 das Membranpotential mißt,
b) die Wirtszellen mit einer Substanz in Kontakt bringt und
c) an Wirtszellen erneut das Meinbranpotential mißt,
wobei der Unterschied zwischen dem Membranpotential vor und nach Zugabe der Substanz die Aktivität der Substanz bestimmt.

30. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet,** daß man
a) an Wirtszellen nach den Ansprüche 11 bis 17 das Membranpotential und den Kaliumauswärtsstrom mißt,
b) die Wirtszellen mit einer Substanz in Kontakt bringt und
c) das Membranpotential und den Kaliumauswärtsstrom erneut mißt,
wobei die Unterschiede zwischen dem Membranpotential und dem Kaliumauswärtsstrom vor und nach Zugabe der Substanz die Aktivität der Substanz bestimmt.

31. Verfahren nach den Ansprüchen 21 bis 30 zur Identifizierung von antibiotisch wirksamen Substanzen.

32. Verfahren nach den Ansprüchen 21 bis 31, dadurch gekennzeichnet, daß man vor Schritt a) testet, ob die Substanzen an bakterielle Kaliumkanalprotein binden, die in prokaryontischen Zellen hergestellt sind.

33. Verwendung der in SEQ ID NO: 1 dargestellten Nukleotidsequenz zur Expression eines bakteriellen Kaliumkanals in eukaryotischen Zellen.

34. Verwendung eines Nukleinsäuremoleküls nach den Ansprüchen 5 bis 7 zur Expression eines bakteriellen Kaliumkanals in eukaryotischen Zellen.
